# EUROPEAN PATENT APPLICATION

(11) **EP 1 952 695 A1**
(43) Date of publication of application: **06.08.2008**
(21) Application number: 07075079.9
(22) Date of filing: 31.01.2007
(51) Int. Cl.: A23D 7/02, A23D 9/02, A23L 1/30, A61K 31/202

(54) **Oil bodies and method of producing such oil bodies**

(71) Applicant: Unilever N.V., 3013 AL Rotterdam (NL)
(72) Inventor: Lemetter, Cedric Yves Ghislain c/o Unilever R&D Vlaardingen BV, 3133 AT Vlaardingen (NL); Beindorff, Christiaan Michaël c/o Unilever R&D Vlaardingen BV, 3133 AT Vlaardingen (NL); Draaisma, René Bernardus c/o Unilever R&D Vlaardingen BV, 3133 AT Vlaardingen (NL); Melnikov, Sergey Michailovich c/o Unilever R&D Vlaardingen BV, 3133 AT Vlaardingen (NL); Castenmiller, Wilhelmus Adrianus M. c/o Unilever R&D Vlaardingen BV, 3133 AT Vlaardingen (NL)
(74) Representative: Joppe, Hermina Laura Petronella

(57) **Abstract**

The present invention relates to a process for obtaining oil bodies, said oil bodies having a volume weighted mean diameter within the range of 0.1-100 µm and comprising a lipid matrix surrounded by a layer of phospholipids embedded with structural proteins, wherein the lipid matrix contains at least 80 wt.% of glycerides, said glycerides containing at least 3 % ω-3 C₂₀-C₂₄ polyunsaturated fatty acid residues (ω-3 LC PUFA) by weight of the total amount of fatty acid residues contained in said glycerides. The invention further relates to oil bodies obtainable by the process of the invention and to compositions comprising at least 0.1 wt.% of these oil bodies.

## Description

### TECHNICAL FIELD OF THE INVENTION

The present invention relates to compositions comprising oil bodies. These oil bodies have a volume weighted mean diameter within the range of 0.1-100 µm and comprise a lipid matrix surrounded by a layer of phospholipids embedded with structural proteins.

The invention also concerns a novel process for the production of oil bodies.

### BACKGROUND OF THE INVENTION

It is well-known in the food industry that the application of oils containing high levels of polyunsaturated fatty acids can give rise to serious off-flavour problems. These off-flavour problems are associated with the oxidation of the polyunsaturated fatty acids, leading to the formation of volatile, potent flavour molecules, such as unsaturated aldehydes. Flavour attributes associated with oxidation products of polyunsaturated fatty acids include "fish", "cardboard", "paint", "oily", "rancid" and "metallic".

Whilst all polyunsaturated fatty acids have been associated with the aforementioned stability problems, lipids containing w-3 polyunsaturated fatty acid residues, notably lipids containing ω-3 C₂₀-C₂₄ polyunsaturated fatty acid residues (ω-3 long chain PUFA or ω-3 LC PUFA) are known to be extremely susceptible to oxidation. Oxidation of the latter fatty acid residues yields volatile oxidation products that introduce a foul "fishy" smell to the product containing these fatty acid residues. Typical examples of ω-3 LC PUFA include docosahexaenoic acid (DHA) and eicosapentaenoic acid (EPA).

Although it is known that the rate of oxidation of polyunsaturated fatty acids may be reduced through the addition of anti-oxidants, the application of such anti-oxidants at best delays the formation of unacceptable off-flavour. However, the shelf-life of products containing appreciable amounts of w-3 LC PUFA, especially food products and beverages, is inherently limited as a result of the inevitable oxidation of these fatty acid residues. Furthermore, in some product applications, e.g. in water-containing products containing dissolved pro-oxidative salts of e.g. iron or copper, it is essentially impossible to employ ω-3 LC PUFA as rapid oxidation of these fatty acid residues will commence as soon as the product has been compounded.

Many, many scientific publications have been issued during the past two decades that strongly suggest that regular consumption of significant amounts of ω-3 LC PUFA can deliver important health benefits. In particular, there is compelling evidence that mental health, (development of) cognitive capabilities and cardiovascular health are positively affected by the consumption of ω-3 LC PUFA. Hence, there has been no lack of effort in the industry to develop food products, beverages, nutritional preparations and pharmaceutical preparations that contain appreciable amounts of ω-3 LC PUFA. However, widespread application of ω-3 LC PUFA in especially food products and beverages has been frustrated by the oxidative instability of these fatty acids.

### SUMMARY OF THE INVENTION

The present inventors have unexpectedly that the oxidative instability of ω-3 LC PUFA in products can be reduced dramatically by incorporating ω-3 LC PUFA in the form of oil bodies and that these oil bodies can be obtained with a special process.

Oil bodies are the discrete intracellular organelles in which triacylglycerides are stored in, for instance, plant seeds. Plant oil bodies are spherical particles about 0.5-4 *µ*m in diameter and are found in all oilseed plants (i.e. canola, flax, sunflower, soybean and corn). The oil-bodies consist primarily of a central droplet of oil (predominantly consisting of triglycerides) that is surrounded by a coat essentially consisting of a phospholipid monolayer and structural proteins.

Oil bodies are remarkably stable both inside cells and in isolated preparations. In both situations the oil bodies occur as individual entities. The structural proteins that together with the phospholipid monolayer surround the central lipid core of the oil bodies, are believed to protect the phospholipids in the monolayer by making them inaccessible to external phospholipase A2 and phospholipase C. Structural proteins found in plant oil bodies include oleosins and caleosins.

The structural protein oleosin is a relatively small protein (15 to 26 kD) and typically contains a central stretch of about 72 hydrophobic residues that is inserted into the lipid core whereas the amphiphatic N-terminal and C-terminal are located at the surface of the oil body. Both terminal portions of the oleosin can be either short (e.g. 4 residues) or very long (e.g. 1200 residues). The central hydrophobic stretch of about 72 uninterrupted hydrophobic residues is the hallmark of an oleosin. No other known protein in any organism has such a long hydrophobic stretch (Hsieh et al., Plant Physiol. 2004 November; 136(3): 3427-3434).

The key structural features of caleosins are an N-terminal region with a single Ca²⁺- binding EF hand domain, a central hydrophobic region able to form a single bilayer span, and a C-terminal region with several putative protein kinase phosphorylation sites (D.J. Murphy, LIPID ASSOCIATED PROTEINS IN PLANTS, 16th Int. Plant Lipid Symposium, Budapest, June 2004, pages 1-8). Caleosins lack an N-terminal signal peptide, but do have a central, hydrophobic region of typically more than 30 residues. Like oleosins, caleosins contain a proline-rich region with the potential to form a "proline know" motif.

The inventors have found that ω-3 LC PUFA containing oil bodies that are naturally present in some micro-organisms, notably algae, may be recovered from these micro-organisms essentially undamaged and that the use of such oil bodies in food products provides the well-sought oxidative stability for ω-3 LC PUFA.

### DETAILED DESCRIPTION OF THE INVENTION

The invention relates to a process for the isolation of oil bodies as defined herein before, said process comprising:
- subjecting micro-organisms containing such oil bodies in a dry form to conditions that will cause rupture of the micro-organism cells;
- optionally adding water, and creating a dispersion of the ruptured cells in water;
- allowing the oil bodies to be released from the cells into the surrounding water; and
- separating the oil bodies from the ruptured cell biomass.

The micro-organisms are in a dry form before they are subjected to conditions that will cause rupture of the micro-organism cells.

According to a particularly preferred embodiment, the micro-organisms employed in the present process are marine micro-organisms, preferably an algae strain belonging to one of the species *Crypthecodinium* cohnii, *Odontella aurita* or an algae strain of a species belonging to the *Thraustochytriidae* group. Most preferably, the algae strain is an algae strain of a species belonging to the *Thraustochytriidae* group.

In the present method, the oil bodies may be separated from the ruptured cell biomass by means of any suitable separation technique. Examples of suitable separation techniques include decanting, filtration and centrifugation. Most preferably, the oil bodies are separated from the ruptured cell biomass by means of centrifugation. In order to facilitate separation of the oil bodies from the ruptured cell biomass, suitably processing agents are added, e.g. to increase the density of the aqueous cell biomass.

Accordingly, one aspect of the invention relates to a composition comprising at least 0.1 wt.% of oil bodies, said oil bodies having a volume weighted mean diameter within the range of 0.1-100 *µ*m and comprising a lipid matrix surrounded by a layer of phospholipids embedded with structural proteins, wherein the lipid matrix contains at least 80 wt.% of glycerides, said glycerides containing at least 3 %, preferably at least 5 % ω-3 C₂₀-C₂₄ polyunsaturated fatty acid residues (ω-3 LC PUFA) by weight of the total amount of fatty acid residues contained in said glycerides.

The term "glyceride" as used herein encompasses lipids selected from the group consisting of triglycerides, diglycerides, monoglycerides, phosphoglycerides and combinations thereof. More preferably, the glycerides are selected from the group consisting of triglycerides and diglycerides. Most preferably, the glyceride is triglyceride oil.

The aforementioned lipid matrix represents the core of the oil body and preferably contains at least 90 wt.% of glycrerides, even more preferably at least 95 wt.% of glycerides and most preferably at least 98 wt.% of glycerides. Besides glycerides of various structures and fatty acid chain length the lipid matrix may also contain other minor components that are dissolved in said matrix.

The composition according to the present invention advantageously comprises 0.3-10 wt.% of the oil bodies. Even more preferably, said composition contains 0.5-8 wt.% of said oil bodies. The volume weighted mean diameter of the present oil bodies typically is within the range of 0.3-50 µm, especially within the range of 0.5-30 µm. The phospholipid layer surrounding the lipid core of the oil body usually is a monolayer.

The oil bodies employed in according with the present invention are advantageously derived from a micro-organism selected from the group consisting of algae, fungi, yeast and combinations thereof. More preferably, the oil bodies are derived from algae or fungi, most preferably from algae. The amino acid sequence of the structural protein in the oil bodies can be used to identify the exact origin of the oil body.

The oil bodies of the present invention are suitably isolated from marine micro-organisms. Members of the following families are capable of producing ω-3 LC PUFA: *Thraustochytriidae, Crypthecodiniaceae, Gymnodiniaceae, Prorocentraceae, Haptophyceae, Skeletonemataceae, Cryptomonadaceae, Phaeodactylaceae.* Specific examples of ω-3 LC PUFA producing species include: *Amphidinium, Amphor, Asterionella, Biddulphia, Ceratium, Chaetoceros, Chlorella, Chroomonas, Cochlodinium, Crisphaera, Crypthecodinium, Cryptomonas, Cyclotella, Cylindrotheca, Duniella, Emiliania, Fragilaria, Glenodinium, Gonyaulax, Gyrodinium, Haematococcus pluvialis, Heteromastix, Isochrysis, Lauderia, Monochrysis, Monodus, Moritella, Mortierella, Nannochloris, Nannochloropis, Navicula, Ni tzschia, Odontella, Olisthodiscus, Pavlova, Peridinium, Phaeodactylum, Porphyridium, Prorocentrum, Pseudopedinella, Rhodella, Rhododomas, Schizochytrium, Skelentonema, Stauroneis, Tetraselmis, Thalassiosira, Thrautochytrium, Ulkenia.*

According to a particularly preferred embodiment, the oil bodies are derived from marine micro-organisms, preferably from an algae strain belonging to one of the species *Crypthecodinium cohnii, Odontella aurita* or an algae strain of a species belonging to the *Thraustochytriidae* group. Most preferably, the algae strain is an algae strain of a species belonging to the *Thraustochytriidae* group. Preferred species belonging to the *Thraustochytriidae* group are *Schizochytrium, Thraustochytrium, Ulkenia, Aplanochytrium, Japonochytrium.*

Lipid containing isolates from algae have been marketed for use in animal feed. These isolates contain appreciable amounts of algae biomass. In accordance with the present invention it is preferred to employ relatively 'clean' oil bodies. Accordingly, in advantageous embodiment, no algae biomass or more preferably no biomass at all, is attached to the oil bodies or such (algae) biomass is attached in an amount of less than 50%, preferably of less than 30% by weight of the oil bodies.

The benefits of the present invention are particularly appreciated when the composition contains a significant amount of water, e.g. at least 1 wt.% of water. Preferably, the present composition contains at least 5 wt.%, more preferably at least 10 wt.% and most preferably at least 15 wt.% of water. In case the present product contains water, the oil bodies are preferably dispersed within the water.

As explained herein before, oxidation of ω-3 LC PUFA is stimulated by the presence of pro-oxidative metal salts. The oil bodies of the present invention display a remarkable oxidative stability even in the presence of such pro-oxidative metal salts. Presence of pro-oxidative metal salts in a product cannot be avoided if tap water or mineral water is used. Furthermore, the incorporation of such metals may be desirable from a nutritional perspective. Hence, according to a preferred embodiment, the water contained in the present product contains at least 5 mg per kg of water, preferably at least 10 mg per kg of water of a pro-oxidative metal cation selected from iron, copper, zinc and combinations thereof. According to a particularly preferred embodiment, the present composition contains at least 0.1 mg/kg or even at least 1 mg/kg of iron cations, more preferably at least 5 mg/kg, even more preferably at least 10 mg/kg and most preferably at least 15 mg/kg of iron cations.

Triglycerides typically constitute the bulk of the oil bodies of the present invention. Typically, the oil bodies contain at least 80 wt.% of triglycerides. More preferably, the oil bodies contain at least 90 wt.% of triglycerides. The triglycerides contained in the lipid matrix preferably contain at least 10%, most preferably at least 20% ω-3 LC PUFA by weight of the total amount of fatty acid residues contained in said triglycerides.

The oil bodies of the present invention can suitably contain a variety of ω-3 LC PUFA, including eicosapentaenoic acid and docosahexaenoic acid. The ω-3 LC PUFA in the present oil bodies is preferably selected from eicosapentaenoic acid (EPA), docosahexaenoic acid (DHA) and combinations thereof. Most preferably, ω-3 LC PUFA is DHA.

In algae oil usually both EPA and DHA are present. Typically, the weight ratio of EPA:DHA in algae oil is in the range of 1:100 to 100:1. In the present oil bodies, the weight ratio of EPA:DHA preferably is in the range of 1:80 to 10:1, more preferably in the range of 1:60 to 1:10.

Phospholipids are normally present in the oil bodies of the present invention in a concentration of 0.2-5 wt.%. The structural proteins usually represent 0.2-7 wt.% of the oil bodies.

The structural protein present in the oil bodies of the present invention preferably has a hydrophilic N-terminal portion and/or a hydrophilic C-terminal portion; and a central stretch of preferably at least 25, more preferably of at least 30, even more preferably at least 50 and most preferably at least 70 uninterrupted hydrophobic residues. According to another preferred embodiment, the central stretch of hydrophobic residues of the structural protein is inserted into the lipid core and the amphiphatic N-terminal and/or amphiphatic C-terminal are located at the surface of the oil bodies, with positively charged residues embedded in a phospholipid monolayer and the negatively charged ones exposed to the exterior. In accordance with a particularly preferred embodiment, the structural protein contains both an amphiphatic N-terminal and an amphiphatic C-terminal with are both located at the surface of the oil bodies. Most preferably, the structural protein employed in accordance with the present invention is an oleosin or a caleosin.

The structural proteins present in the oil bodies of the present invention typically have a molecular size of 10-100 kD, more preferably of 12-70 kD.

The present invention enables the application of ω-3 LC PUFA in a wide array of food products. Thus, in an advantageous embodiment, the composition is an edible composition, such as a food product or a beverage. Even more preferably, the composition is selected from the group consisting of beverages (e.g. nutritional beverages and dairy drinks), spreads, nutrition bars, pasta products, ice cream, desserts, dairy products (e.g. yogurt, quark, cheese), dressings, sauces, soups, instant powders, fillings, dips and breakfast type cereal products (e.g. porridge). According to a particularly preferred embodiment, the edible product is a beverage, a nutrition bar or an instant powder. The term "instant powder" refers to an essentially dry powder that prior to consumption can be reconstituted with water to produce, for instance, a beverage or a soup.

The edible composition of the present invention may suitably contain additional ingredients such as carbohydrates, emulsifiers, herbs, spices, anti-oxidants, preservatives, flavourings, colourings, vitamins and minerals. Furthermore, the edible composition may optionally comprise, in suitable amounts, one or more agents which may beneficially influence (post-prandial) energy metabolism and substrate utilisation, for example caffeine and flavonoids (including tea catechins, capsaicinoids and canitine).

According to a preferred embodiment, the edible composition contains added vitamins selected from at least one of: Vitamin A, Vitamin B1, Vitamin B2 (riboflavin), Vitamin B3 (Niacinamide), Vitamin B5 (d-Ca-pantothenate), Vitamin B6, Vitamin B11, Vitamin B12 (Cyanocobalamin), Vitamin C (Ascorbic acid), Vitamin D, Vitamin E (Tocopherol),Vitamin H (Biotin), and Vitamin K.

The composition also preferably comprises added minerals selected from at least one of; calcium, magnesium, potassium, zinc, iron, cobalt, nickel, copper, iodine, manganese, molybdenum, phosphorus, selenium and chromium.

One or more of the above-mentioned vitamins and minerals are preferably present at amounts of from 5 to 45% of the amounts given in the above European Commission Directive 96/8/EC, especially 5 to 40%, most especially 10 to 30%.

Another aspect of the present invention relates to the use of the oil bodies as defined herein before in the treatment or prevention of disorders or of impaired cognitive function. Examples of disorders that may suitably be treated with the present oil bodies include atherosclerosis, cardiovascular disorders and coronary heart disease. The ω-3 LC PUFA in the oil bodies helps to reduce or prevent serum oxidative stress leading to the aforementioned disorders. The present oil bodies are also advantageously used in the improvement or treatment of cognitive and mental conditions and disorders as well as the maintenance of normal functions of brain-related systems and processes, preferably ADHD, aging, Alzheimer's disease, Parkinson's disease, multiple sclerosis (MS), dyslexia, depression, learning capabilities, stress, anxiety, concentration and attention.

The aforementioned uses preferably comprise oral administration of the oil bodies. Typically, the oil bodies are administered in an amount that is sufficient to deliver at least 10 mg of ω-3 LC PUFA per administration event. More preferably, at least 30 mg, even more preferably at least 50 mg and most preferably at 100 mg of ω-3 LC PUFA is delivered per administration event.

As will be appreciated from the above, the oil bodies are advantageously ingested in the form of an edible product, e.g. a food product, a beverage, a nutritional preparation or a pharmaceutical preparation. Most preferably, the edible product is a food product or a beverage. It will be understood that the present invention may suitably be employed to treat animals, such as mammals. Most preferably, the present invention is used to treat humans.

The invention is further illustrated by means of the following examples.

### EXAMPLES

### Example 1

Oil bodies were extracted from dry *Schizochytrium* micro-algae biomass. The composition of the starting material is presented in Table 1:

**Table 1**

| | **Wt.%** |
|---|---|
| Inorganic matter | 8.2 |
| Fat # | 59.4 |
| Protein | 2.5 |
| Moisture | 11.4 |
| Carbohydrates * | 18.5 |

| | |
|---|---|
| * Value estimated by subtraction of 100% minus protein, fat, inorganic matter and water content. # Soxhlet extraction of 80 grams of dried algae with hexane, followed by hexane removal in a rotary evaporator, yielded 19.2 grams of extracted oil, said oil having a fatty acid composition containing 0.05% α-linolenic acid (ALA), 1.32% EPA and 34.72% DHA. | |

The oil bodies were extracted from dry algae biomass using the following procedure. Fifty gram of dry *Schizochytrium* micro-algae biomass was mixed with 500 ml of grinding solution (0.6M sucrose) in a Waring blender at a speed of 22,000 rpm for one minute. The extraction solution, which contained the oil bodies and different solutes, was separated from debris using a laboratory sieve with a mesh size of 150 µm.

The liquid phase was further processed in a Sorvall centrifuge, equipped with a GSA rotor, for 30 minutes at 8,000 rpm in order to isolate the oil bodies. A separation in three phases was observed. At the bottom a layer of sediment, consisting mainly of fibres and proteins was formed. In the middle an aqueous phase consisting of water and solutes was formed. The top layer consisted of a creamy phase consisting of oil bodies mixed with water and some impurities.

The cream top layer was carefully removed with a spoon and subjected to three more liquid-liquid separation steps successively using three different aqueous buffer solutions 1, 2 and 3.

| | |
|---|---|
| Floating buffer 1 | 0.2M sucrose / 1.0M NaCl |
| Floating buffer 2 | 0.1M sucrose / 1.0M NaCl |
| Floating buffer 3 | 0.2M NaCl |

The composition of the *Schizochytrium* micro-algae oil bodies was determined for three different batches of oil bodies. The results are presented in Table 2:

**Table 2: Composition of three different batches of Schizochytrium micro-algae oil bodies.**

| | | **Batch 1** | **Batch 2** | **Batch 3** |
|---|---|---|---|---|
| Fat (wt%) | | 24.9 | 16.1 | -- |
| Moisture (wt%) | | 68.9 | 72.8 | 84.7 |
| Protein (wt%) | | -- | 1.9 | -- |
| Particle size distribution* | D₃, ₂ (*µ*m) | 4.4 | 9.5 | 4.8 / 4.8 |
| | D₄, ₃ (*µ*m) | 7.8 | 20.4 | 9.7 / 10.1 |

| | | | | |
|---|---|---|---|---|
| * D_{3, 2} is the surface-weighted mean diameter and D_{4, 3} is the volume-weighted mean diameter. | | | | |

Both the isolated oil bodies as well as the algae starting material were analysed by means of confocal scanning laser microscopy (CSLM). Microscopic observations were performed on a Bio-Rad confocal microscope equipped with a Zeiss inverted microscope system using an oil immersion 40 times objective lens N.A. 1.25. The Bio-Rad system was controlled by Lasersharp software running under OS2. Two laser lines from the argon / krypton laser, 488 and 647 nm were used to excite the fluorochrome nile blue. Green fluorescence (excitation wavelength 488 nm) indicating the presence of lipids was detected in a band at 605 +/- 16 nm. The red fluorescence (excitation wavelength 647 nm) indicating the presence of protein and other more polar components was detected at 680 +/-16 nm.

*Schizochytrium* micro-algae biomass and the isolated oil body suspensions isolated therefrom were coloured with a nile blue solution. On both the CSLM images of the *Schizochytrium* micro-algae and the oil body suspensions, green spheres were seen. In the CSLM images of the micro-algae biomass, substantial parts of the green spheres were coloured red, indicating that protein cell-wall material is attached to the oil core.

### Example 2

In order to investigate the oxidative stability of oil bodies an accelerated storage test was performed. The stability of the oil contained in oil bodies obtained through the isolation procedure described in Example 1 was compared with the oxidative stability of commercially available pure oil isolated from the same *Schizochytrium* source (ex Martek Biosciences, USA). Unlike the isolated oil bodies, the pure algae oil contained added anti-oxidants (rosemary extract, tocopherols and ascorbyl palmitate).

In a series of serum flasks were added: 16.7 gram of oil bodies of batch 1 of Example 1 or a comparable amount of the same type of pure oil (5.0 gram), 0.140 g of Hymono and 60 ml of a 0.035 wt% citric acid buffer pH 5.0. The flasks was flushed with air for five minutes to ensure that the headspace contained 20.9% oxygen. To minimise light-induced oxidation of the emulsions, the flasks were wrapped in aluminium foil, incubated in an incubator shaker (Innova 4230, New Brunswick Scientific, USA) at 60 °C, and shaken at 250 rpm for a maximum of 96 hours. At time intervals of 24 hours, oxygen headspace analysis (Servomex 574 provided with Digitron pressure indicator P200AH) was performed on headspace samples taken from the flasks.

The results obtained are depicted in Table 3:

**Table 3**

| | Oxygen concentration after | | | | |
|---|---|---|---|---|---|
| | 0 hr | 24 hrs | 48 hrs | 72 hrs | 96 hrs |
| Pure oil | 20.9 | 19.8 | 15.3 | 6.8 | 0.1 |
| Oil bodies | 20.9 | 19.9 | 19.2 | 18.6 | 18.1 |

These results clearly show that much less oxygen is consumed by sample containing the oil bodies than the by the sample containing the pure oil.

In addition, off-flavour formation after accelerated storage was analysed: Approximately 20 gram emulsion was distributed among 20 headspace vials (Chrompack, 20 ml). In all samples the amount of fat was kept constant. The sample vials were flushed with 100% oxygen, capped and placed in an oven at 60 °C in the dark. The samples are oxidised in the oven without stirring or mechanical agitation. Duplicate samples were removed from the oven at regular time intervals (typically twice a day) and cooled down to ambient temperature in the dark. The samples were de-capped and flushed for ca. 6 seconds with a nitrogen stream at a flow rate of 500 ml/min to prevent further oxidation. The samples were re-capped and stored at -18 °C until further analysis.

Samples oxidised for various lengths of time were collected for headspace analysis using a GC-17A gas chromatograph (Shimadzu). The samples were placed in an automated headspace sampler at 40 °C and allowed to equilibrate for at least 30 minutes. Headspace samples (2.5 ml) were taken with a gas-tight syringe and injected in split mode (ratio 5:1) onto a capillary CP-Sil 5CB column (Chrompack). Column specifications: length 25 m, inside diameter 0.25 mm, outside diameter 0.39 mm and film thickness 1.20 µm. The chromatograph was equipped with a flame ionisation detector. Temperatures at the inlet and detector were 200 °C and 280 °C, respectively. The temperature programming for gas chromatographic analysis was: 0 °C (4 min) - 40 °C/min - 40 °C (0 min) - 5 °C/min - 60 °C (0 min) - 40 °C/min - 200 °C (3 min). Samples were measured in random order to avoid systematic errors due to e.g. carry over effects. Peak areas (in *µ*V.s) were quantified for the following oxidation off-flavours: acetaldehyde, propenal, pentane, 1-penten-3-one, 1-penten-3-ol, pentanal, trans-2-pentenal and hexanal. Peak areas of duplicate samples were averaged and plotted as a function of storage time at 60 °C. The results obtained are presented in Tables 4-11.

**Table 4**

| | Acetaldehyde concentration (in µV.s) after | | | | | |
|---|---|---|---|---|---|---|
| | 0 hr | 16 hrs | 24 hrs | 40 hrs | 48 hrs | 64 hrs |
| Pure oil | 715 | 3380 | 9837 | 49410 | 61874.5 | 698091 |
| Oil bodies | 661.5 | 9038.5 | 8122 | 30573 | 44241.5 | 141770 |

**Table 5**

| | Propenal concentration (in µV.s) after | | | | | |
|---|---|---|---|---|---|---|
| | 0 hr | 16 hrs | 24 hrs | 40 hrs | 48 hrs | 64 hrs |
| Pure oil | 888 | 4500.5 | 5932.5 | 44065 | 78036.5 | 1573386 |
| Oil bodies | 0 | 2121 | 1994 | 3238.5 | 3221 | 10104 |

**Table 6**

| | Pentane concentration (in µV.s) after | | | | | |
|---|---|---|---|---|---|---|
| | 0 hr | 16 hrs | 24 hrs | 40 hrs | 48 hrs | 64 hrs |
| Pure oil | 743 | 395.5 | 564 | 6412 | 4662 | 198360 |
| Oil bodies | 9879.5 | 3824.5 | 5757.5 | 4335 | 3969 | 7940.5 |

**Table 7**

| | 1-Penten-3-one concentration (in *µ*V.s) after | | | | | |
|---|---|---|---|---|---|---|
| | 0 hr | 16 hrs | 24 hrs | 40 hrs | 48 hrs | 64 hrs |
| Pure oil | 100.5 | 16 | 9 | 629 | 648.5 | 2695.5 |
| Oil bodies | 13.5 | 18 | 112.5 | 365 | 372 | 816.0 |

**Table 8**

| | 1-Penten-3-ol concentration (in µV.s) after | | | | | |
|---|---|---|---|---|---|---|
| | 0 hr | 16 hrs | 24 hrs | 40 hrs | 48 hrs | 64 hrs |
| Pure oil | 110 | 97 | 128.5 | 1858 | 3606.5 | 184499 |
| Oil bodies | 135.5 | 320.5 | 324 | 1182 | 1140 | 6274 |

**Table 9**

| | Pentanal concentration (in µV.s) after | | | | | |
|---|---|---|---|---|---|---|
| | 0 hr | 16 hrs | 24 hrs | 40 hrs | 48 hrs | 64 hrs |
| Pure oil | 2277 | 1839.5 | 2069.5 | 7627.5 | 9942.5 | 55320 |
| Oil bodies | 250.5 | 4765 | 1920 | 8671 | 10295 | 33028 |

**Table 10**

| | Tr-2-pentenall concentration (in *µ*V.s) after | | | | | |
|---|---|---|---|---|---|---|
| | 0 hr | 16 hrs | 24 hrs | 40 hrs | 48 hrs | 64 hrs |
| Pure oil | 10 | 142 | 225.5 | 3364.5 | 5160.5 | 111503 |
| Oil bodies | 11.5 | 152 | 140.5 | 1134.5 | 1219.5 | 6615 |

**Table 11**

| | Hexanal concentration (in *µ*V.s) after | | | | | |
|---|---|---|---|---|---|---|
| | 0 hr | 16 hrs | 24 hrs | 40 hrs | 48 hrs | 64 hrs |
| Pure oil | 799 | 2402.5 | 5633 | 19100.5 | 19100.5 | 266354 |
| Oil bodies | 258.5 | 3651.5 | 2080.5 | 2080.5 | 2080.5 | 113038 |

These results show clearly that the formation of secondary oxidation products is decreased in *Schizochytrium* micro-algae oil bodies in comparison to the pure algae oil. This observation is in-line with the above mentioned results from the accelerated storage test. The combined results of these tests demonstrate that *Schizochytrium* micro-algae oil bodies have a better oxidative stability than the pure algae oil. The aforementioned findings are truly remarkable since the pure algae oil contained added anti-oxidants, whereas the oil bodies did not.

## Claims

1. Process for the isolation of oil bodies said oil bodies having a volume weighted mean diameter within the range of 0.1-100 µm and comprising a lipid matrix surrounded by a layer of phospholipids embedded with structural proteins, wherein the lipid matrix contains at least 80 wt.% of glycerides, said glycerides containing at least 3 %, preferably at least 5 % ω-3 C₂₀-C₂₄ polyunsaturated fatty acid residues by weight of the total amount of fatty acid residues contained in said glycerides, said process comprising:
- subjecting dry micro-organisms containing such oil bodies to conditions that will cause rupture of the micro-organism cells;
- optionally adding water, and creating a dispersion of the ruptured cells in water;
- allowing the oil bodies to be released from the cells into the surrounding water; and
- separating the oil bodies from the ruptured cell biomass.

2. Process according to claim 1, wherein the micro-organism is a marine micro-organism.

3. Process according to claim 2 wherein the marine micro-organism is an algae strain selected from the group consisting of *Crypthecodinium cohnii, Odontella* aurita and *Thraustochytriidae.*

4. Process according to any of claim 1 to 3, wherein the oil bodies are separated from the ruptured cell biomass by means of centrifugation.

5. Oil bodies obtainable by a process according to claim 1 to 4.

6. A composition comprising at least 0.1 wt.% of oil bodies, said oil bodies having a volume weighted mean diameter within the range of 0.1-100 µm and comprising a lipid matrix surrounded by a layer of phospholipids embedded with structural proteins, wherein the lipid matrix contains at least 80 wt.% of glycerides, said glycerides containing at least 3 %, preferably at least 5 % ω-3 C₂₀-C₂₄ polyunsaturated fatty acid residues by weight of the total amount of fatty acid residues contained in said glycerides wherein no biomass is attached to the oil bodies or wherein such biomass is attached in an amount of less than 50%, preferably of less than 30% by weight of the oil bodies.

7. Composition according to claim 6, wherein the composition contains at least 1 wt.% of water and the oil bodies are dispersed within the water.

8. Composition according to claim 7, wherein the water contains at least 5 mg per kg of water of a pro-oxidative metal cation selected from iron, copper, zinc and combinations thereof.

9. Composition according to claim 8, wherein the composition contains at least 5 mg/kg of iron.

10. Composition according to any one claim 6 to 9, wherein the oil bodies are derived from a marine micro-organism.

11. Composition according to claim 10 wherein the marine micro-organism is selected from the group consisting of *Crypthecodinium cohnii, Odontella* aurita and *Thraustochytriidae.*

12. Composition according to any one claim 6 to 11, wherein the composition is a food product or a beverage.

13. Composition according to any one claim 6 to 12, wherein the structural protein has a hydrophilic N-terminal portion and/or a hydrophilic C-terminal portion; and a central stretch of at least 25, preferably at least 30 uninterrupted hydrophobic residues.

14. Composition according to claim 13, wherein the structural protein is an oleosin or a caleosin.

15. Use of the oil bodies as defined in claim 5 or the composition as defined in claim 6 in the manufacture of a composition for use in the treatment of prevention of disorders or impaired cognitive function.

16. Use of the oil bodies as defined in claim 5 or the composition as defined in claim 6 in the manufacture of a composition for use in the reduction or prevention of atherosclerosis, cardiovascular disorders or coronary heart disease.

17. Use according to claim 15 in the improvement or treatment of cognitive and mental conditions and disorders as well as the maintenance of normal functions of brain-related systems and processes, preferably ADHD, aging, Alzheimer's disease, Parkinson's disease, multiple sclerosis (MS), dyslexia, depression, learning capabilities, stress, anxiety, concentration and attention.

18. Use according to any one of claims 15-17, wherein the use comprises oral administration of the oil bodies in an amount that is sufficient to deliver at least 10 mg of ω-3 C₂₀-C₂₄ polyunsaturated fatty acid residues per administration event.
